# EUROPEAN PATENT SPECIFICATION

(11) **EP 0 426 468 B1**
(45) Date of publication and mention of the grant of the patent: **06.09.1995**
(21) Application number: 90311963.4
(22) Date of filing: 01.11.1990
(51) Int. Cl.: C07C 255/41

(54) **Stabile polymorphic form of (E)-N,N-diethyl-2-cyano-3-(3,4-dihydroxy-5-nitrophenyl) acrylamide and the process for its preparation**
Stabile polymorphe Form von (E)-N,N-Diethyl-2-cyano-3-(3,4-dihydroxy-5-nitrophenyl)-Acrylamid und Verfahren zu ihrer Herstellung
Forme polymorphe stable de (E)-N,N-diéthyl-2-cyano-3-(3,4-dihydroxy-5-nitrophényl) acrylamide et procédé pour sa préparation

(30) Priority: 03.11.1989 GB 8924838
(43) Date of publication of application: 08.05.1991
(73) Proprietor: ORION-YHTYMÄ OY, 02200 Espoo (FI)
(72) Inventor: Pippuri, Aino Kyllikki, F-02260 Espoo (FI); Honkanen, Erkki Juhani, F-01400 Vantaa (FI); Haarala, Jorma Veikko, F-00720 Helsinki (FI)
(74) Representative: Sexton, Jane Helen

(56) References cited:
- FR-A- 2 607 493

## Description

The present invention relates to the stabile and crystallografically essentially pure polymorphic form of N,N-diethyl-2-cyano-3-(3,4-dihydroxy-5-nitrophenyl)acrylamide E-isomer, denoted (E)-N,N-diethyl-2-cyano-3-(3,4-dihydroxy-5-nitrophenyl)acrylamide A, and to a process for the preparation thereof.

N,N-Diethyl-2-cyano-3-(3,4-dihydroxy-5-nitrophenyl)acrylamide described in GB-A-2200109 is a potent inhibitor of catechol-O-methyl-transferase enzyme (COMT) and may be used pharmaceutically in the treatment of e.g. Parkinson disease. This compound with melting point 153-156°C has proved to be a mixture of two geometric isomers; E- and Z-isomers (70-80% E-isomer and 30-20% Z-isomer) having formulae: 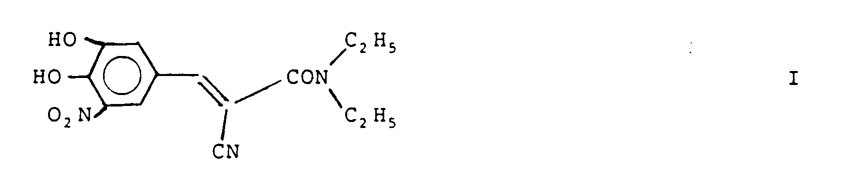
E-isomer, m.p. 162 - 163°C 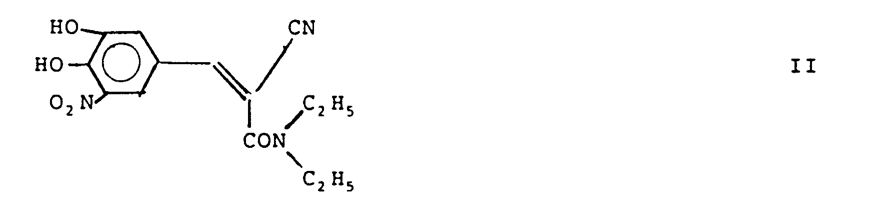
Z-isomer, m.p. 148 - 151°C (E)-N,N-Diethyl-2-cyano-3-(3,4-dihydroxy-5-nitrophenyl)acrylamide (I) may exist at least in two polymorphic forms A and B as shown by X-ray crystallography. The Z-isomer as well as the polymorphic form B of the E-isomer have been shown to be unstabile. The Z-isomer is transformed readily into the E-isomer on the influence of heat or acids. Similarily the polymorphic form B of the E-isomer isomerises slowly to the polymorphic form A on standing at room temperature. On recrystallization of the crude synthesis product from conventional solvents such as lower aliphatic alcohols, esters or hydrocarbons, e.g. ethanol, 2-propanol, ethyl acetate or toluene, a very complicated mixture of different geometric isomers and/or polymorphic forms are generally obtained which interfere with the characterization and standardization of the drug substance. The polymorphism and geometrical isomerism may also influence on the bioavailability of the drug.

Surprisingly it has now been observed that crystallographically essentially pure and stabile polymorphic form A of (E)-N,N-diethyl-2-cyano-3-(3,4-dihydroxy-5-nitrophenyl)acrylamide is obtained in good yield, when the crude product of synthesis is recrystallized from a lower aliphatic carboxylic acid such as formic or acetic acid with a catalytic amount of hydrochloric or hydrobromic acid added. This method allows large scale production of homogenous and crystallographically essentially pure polymorphic form A of (E)-N,N-diethyl-2-cyano-3-(3,4-dihydroxy-5-nitrophenyl)acrylamide independent of batch size or cooling rate.

"Crystallographically essentially pure" means here the polymorphic form A of (E)-N,N-diethyl-2-cyano-3-(3,4-dihydroxy-5-nitrophenyl)acrylamide containing a maximum of 3% and preferably a maximum of 2% of other polymorphic forms or Z-isomer.

"Lower aliphatic carboxylic acid" means here aliphatic carboxylic acid having 1-2 carbon atoms. Examples are formic and acetic acid.

The amount of hydrochloric or hydrobromic acid appropriate for use in crystallising the crude form of the product will vary depending on the exact proportion of isomers in the crude form. Typically hydrobromic acid will be used in a weight ratio of hydrobromic acid: carboxylic acid of 1:80 to 1:120, preferably about 1:100. Typically hydrochloric acid will be used in a weight ratio of hydrochloric acid: carboxylic acid of 1:180 to 1:220, preferably about 1:200.

The crystallization from the crude product takes place preferably by first admixing the crude product and the carboxylic acid and the hydrohalic acid in any order. Most conveniently the hydrohalic acid is first admixed with the carboxylic acid and the crude product then added, although other processes, e.g. the simultaneous admixing of the three components, is also envisaged.

The crude product is preferably used in a weight ratio of crude product to carboxylic acid of 1:1.5 to 1:4 more preferably 1:2 to 1:3.

The mixture is generally heated, preferably with stirring, to a temperature at which the crude product dissolves but below the boiling point of the mixture. Typically this is 80 to 98°C e.g. about 90°C. The solution is then slowly cooled to about 15-20°C and the crystalline product, essentially pure polymorphic form A, may be filtered and washed.

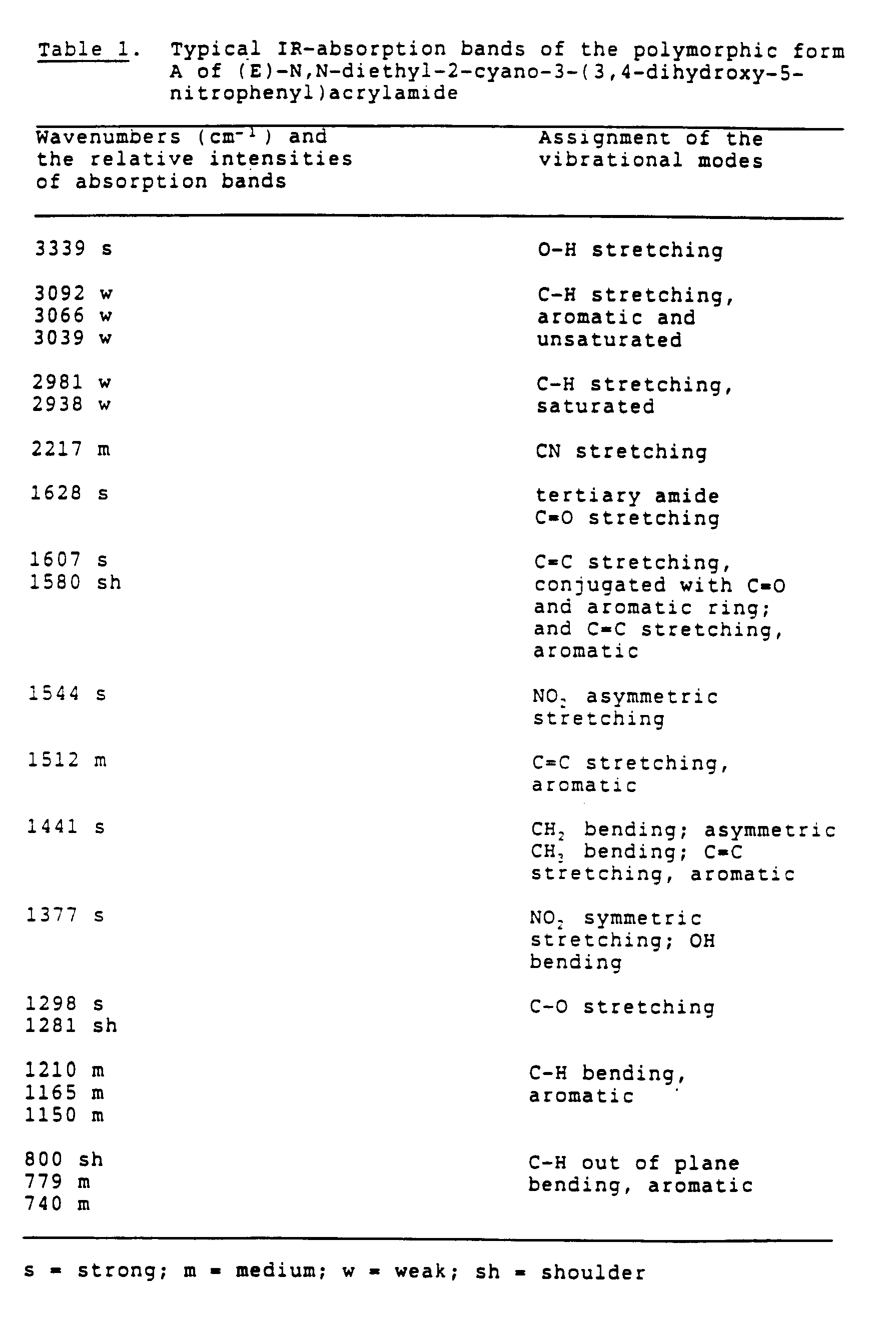

### Experimental

| | |
|---|---|
| Instrument: | Perkin-Elmer FTIR 1725X |
| Detector: | TGS |
| Ordinate mode: | %T |
| Abscissa mode: | Wavenumbers (cm⁻¹) |
| Resolution: | 4 cm⁻¹ |
| Number of scans: | 20 |
| Phase | KBr |

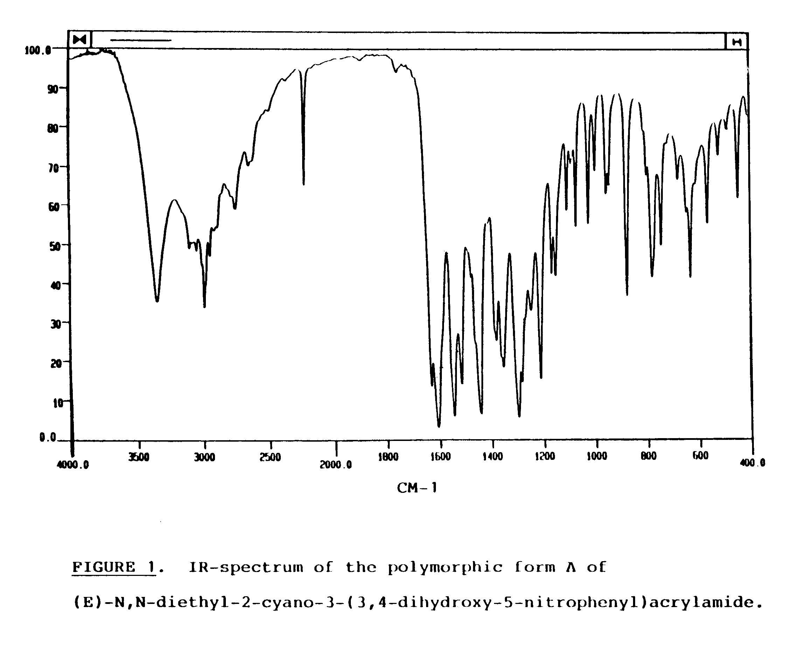

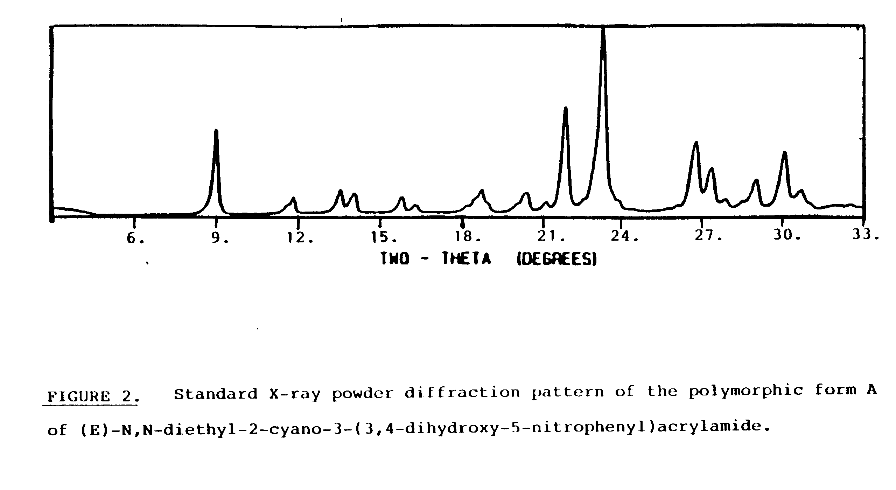

The X-ray powder diffraction patterns of the polymorphic form A of (E)-N,N-diethyl-2-cyano-3-(3,4-dihydroxy-5-nitrophenyl)acrylamide are seen in Figure 2 and the crystallographic data in Table 2.

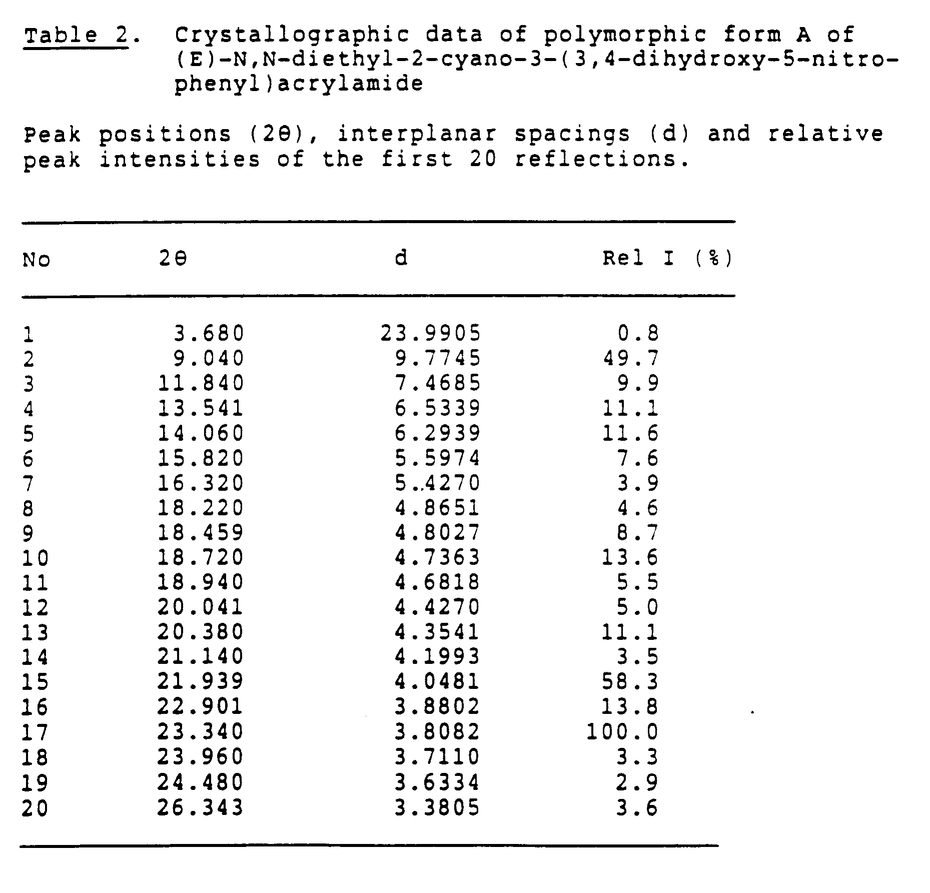

### Experimental

| | |
|---|---|
| Instrument: | Siemens D500 |
| Wavelength: | 0.1541 nm (CuKα₁) |
| Range: | 3°- 33° (2ϑ) |
| Power: | 40 mA/40 kV |
| Time: | 1°/min (0.02°/step) |

The following example illustrates the invention.

### Example 1.

The crude synthesis product (3.0 kg) prepared according to the method described in GB-A-2200109 was dissolved in 8.0 kg of acetic acid (98-100%) (or formic acid) containing 80 g of hydrogen bromide (or 40 g of hydrogen chloride) by heating to 90°C. The solution was slowly cooled to 20°C and stirred for 20 h at 20°C and finally for 6 h at 15°C. The crystalline product was filtered and washed carefully first with a cold (4°C) mixture (1 l) of toluene-acetic acid (1:1 v/v) and then with cold toluene (1 l). The product was dried in vacuum at 45°C. Yield of crystallografically pure A form of (E)-N,N-diethyl-2-cyano-3-(3,4-dihydroxy-5-nitrophenyl)acrylamide was 2.4 kg (80%), m.p. 162-163°C.

## Claims (Claims for the following Contracting State(s): AT, BE, CH, DE, DK, FR, GB, IT, LI, LU, NL, SE)

1. The crystallographically essentially pure polymorphic form A of (E)-N,N-diethyl-2-cyano-3-(3,4-dihydroxy-5-nitrophenyl)acrylamide characterized by the infrared spectrum in potassium bromide having the following absorption bands:
| Wavenumbers (cm⁻¹) | Wavenumbers (cm⁻¹) |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |

2. A process for preparing the crystallographically essentially pure polymorphic form A of (E)-N,N-diethyl-2-cyano-3-(3,4-dihydroxy-5-nitrophenyl)acrylamide of claim 1 which comprises crystallization of crude N,N-diethyl-2-cyano-3-(3,4-dihydroxy-5-nitrophenyl)acrylamide from an aliphatic carboxylic acid having 1 or 2 carbon atoms containing a catalytic amount of hydrochloric or hydrobromic acid.

3. The process as claimed in claim 2, wherein said aliphatic carboxylic acid is acetic acid.

4. The process as claimed in claim 2, wherein said aliphatic carboxylic acid is formic acid.

## Claims (Claims for the following Contracting State(s): ES, GR)

1. A process for preparing the crystallographically essentially pure polymorphic form A of (E)-N,N-diethyl-2-cyano-3-(3,4-dihydroxy-5-nitrophenyl)-acrylamide characterized by the infrared spectrum in potassium bromide having the following absorption bands:
| WAVENUMBERS (cm⁻1) | WAVENUMBERS (cm⁻¹) |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |
which comprises crystallization of crude N,N-diethyl-2-cyano-3 (3,4-dihydroxy-5-nitrophenyl)acrylamide from an aliphatic carboxylic acid having 1 or 2 carbon atoms containing a catalytic amount of hydrochloric or hydrobromic acid.

2. The process as claimed in claim 1, wherein said aliphatic carboxylic acid is acetic acid.

3. The process as claimed in claim 1, wherein said aliphatic carboxylic acid is formic acid.

## Patentansprüche (Patentansprüche für folgende(n) Vertragsstaat(en): AT, BE, CH, DE, DK, FR, GB, IT, LI, LU, NL, SE)

1. Kristallographisch im wesentlichen reine polymorphe Form von (E)-N,N-Diethyl-2-cyano-3-(3,4-dihydroxy-5-nitrophenyl)-acrylamid, dadurch gekennzeichnet, daß das Infrarot-Spektrum in Kaliumbromid die folgenden Absorptionsbanden aufweist:
| Wellenzahlen (cm**⁻¹**) | Wellenzahlen (cm**⁻¹**) |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |

2. Verfahren zur Herstellung der kristallographisch im wesentlichen reinen polymorphen Form von (E)-N,N-Diethyl-2-cyano-3-(3,4-dihydroxy-5-nitrophenyl)-acrylamid nach Anspruch 1, welches die Kristallisation von rohem N,N-Diethyl-2-cyano-3-(3,4-dihydroxy-5-nitrophenyl)acrylamid aus einer aliphatischen Carbonsäure mit 1 oder 2 Kohlenstoffatomen umfaßt, die eine katalytische Menge an Chlorwasserstoff- oder Bromwasserstoffsäure enthält.

3. Verfahren nach Anspruch 2, worin die genannte aliphatische Carbonsäure Essigsäure ist.

4. Verfahren nach Anspruch 2, worin die genannte aliphatische Carbonsäure Ameisensäure ist.

## Patentansprüche (Patentansprüche für folgende(n) Vertragsstaat(en): ES, GR)

1. Verfahren zur Herstellung der kristallographisch im wesentlichen reinen polymorphen Form von (E)-N,N-Diethyl-2-cyano-3-(3,4-dihydroxy-5-nitrophenyl)-acrylamid, dadurch gekennzeichnet, daß das Infrarot-Spektrum in Kaliumbromid die folgenden Absorptionsbanden aufweist:
| Wellenzahlen (cm**⁻¹**) | Wellenzahlen (cm**⁻¹** |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |
welches die Kristallisation von rohem N,N-Diethyl-2-cyano-3-(3,4-dihydroxy-5-nitrophenyl)acrylamid aus einer aliphatischen Carbonsäure mit 1 oder 2 Kohlenstoffatomen umfaßt, die eine katalytische Menge an Chlorwasserstoff- oder Bromwasserstoffsäure enthält.

2. Verfahren nach Anspruch 1, worin die genannte aliphatische Carbonsäure Essigsäure ist.

3. Verfahren nach Anspruch 1, worin die genannte aliphatische Carbonsäure Ameisensäure ist.

## Revendications (Revendications pour l'(les) Etat(s) contractant(s) suivant(s): AT, BE, CH, DE, DK, FR, GB, IT, LI, LU, NL, SE)

1. Forme polymorphique A essentiellement pure du point de vue cristallographique, de (E)-N,N-diéthyl-2-cyano-3-(3,4-dihydroxy-5-nitrophényl)acrylamide, caractérisée par un spectre infrarouge dans le bromure de potassium, présentant les bandes d'absorption suivantes :
| Nombres d'onde (cm⁻¹) | Nombres d'onde (cm⁻¹) |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |

2. Procédé pour la préparation d'une forme polymorphique A essentiellement pure du point de vue cristallographique, de (E)-N,N-diéthyl-2-cyano-3-(3,4-dihydroxy-5-nitrophényl)acrylamide selon la revendication 1, qui comprend la cristallisation du (E)-N,N-diéthyl-2-cyano--3-(3,4-dihydroxy-5-nitrophényl)acrylamide brut à partir d'un acide carboxylique aliphatique comportant 1 ou 2 atomes de carbone contenant une quantité catalytique d'acide chlorhydrique ou d'acide bromhydrique.

3. Procédé selon la revendication 2, dans lequel ledit acide carboxylique aliphatique est l'acide acétique.

4. Procédé selon la revendication 2, dans lequel ledit acide carboxylique aliphatique est l'acide formique.

## Revendications (Revendications pour l'(les) Etat(s) contractant(s) suivant(s): ES, GR)

1. Procédé pour la préparation de la forme polymorphique A essentiellement pure du point de vue cristallographique, de (E)-N,N-diéthyl-2-cyano-3-(3,4-dihydroxy-5-nitrophényl)acrylamide, caractérisée par un spectre infrarouge dans le bromure de potassium, présentant les bandes d'absorption suivantes :
| Nombres d'onde (cm⁻¹) | Nombres d'onde (cm⁻¹) |
|---|---|
| 3339 | 1512 |
| 3092 | 1441 |
| 3066 | 1377 |
| 3039 | 1298 |
| 2981 | 1281 |
| 2938 | 1210 |
| 2217 | 1165 |
| 1628 | 1150 |
| 1607 | 800 |
| 1580 | 779 |
| 1544 | 740 |
qui comprend la cristallisation du (E)-N,N-diéthyl-2-cyano--3-(3,4-dihydroxy-5-nitrophényl)acrylamide brut à partir d'un acide carboxylique aliphatique comportant 1 ou 2 atomes de carbone contenant une quantité catalytique d'acide chlorhydrique ou d'acide bromhydrique.

2. Procédé selon la revendication 1, dans lequel ledit acide carboxylique aliphatique est l'acide acétique.

3. Procédé selon la revendication 1, dans lequel ledit acide carboxylique aliphatique est l'acide formique.
